# EUROPEAN PATENT APPLICATION

(11) **EP 4 328 920 A1**
(43) Date of publication of application: **28.02.2024**
(21) Application number: 22790777.1
(22) Date of filing: 22.03.2022
(51) Int. Cl.: G16B 30/00

(54) **MICROSATELLITE INSTABILITY DETECTION METHOD BASED ON SECOND-GENERATION SEQUENCING**

(30) Priority: 20.04.2021 CN 202110427488
(71) Applicant: Amoy Diagnostics Co., Ltd, Xiamen, Fujian 361027 (CN)
(72) Inventor: LIN, Chenghong, Xiamen City, Fujian 361027 (CN); CHEN, Shaohong, Xiamen City, Fujian 361027 (CN); LI, Xuchao, Xiamen City, Fujian 361027 (CN); JIN, Baolei, Xiamen City, Fujian 361027 (CN); ZHANG, Xiamei, Xiamen City, Fujian 361027 (CN); DONG, Hua, Xiamen City, Fujian 361027 (CN); RUAN, Li, Xiamen City, Fujian 361027 (CN); ZHENG, Limou, Xiamen City, Fujian 361027 (CN)
(74) Representative: Hoppe, Georg Johannes
(86) International application number: PCT/CN2022/082252
(87) International publication number: WO 2022/222668

(57) **Abstract**

A microsatellite instability detection method based on second-generation sequencing. The method comprises the following steps: (1) merging sequencing data of different MSI negative samples, so as to obtain a reference set; (2) compiling statistics on the depths Di of MSI sites i in the reference set; (3) calculating a data feature Fᵢ of a microsatellite sequence of the MSI sites i of the MSI negative samples; (4) compiling statistics, in the entire reference set, on the number of the depths Di of MSI sites i that have a value lower than a threshold value a, and then performing selection and constructing a hybrid reference set; (5) performing, in groups, quality control on the MSI negative samples; (6) performing a first instance of detection; (7) if the number of sites j that are determined to be MSS candidate sites during the first instance of detection is less than d, taking a result of the first instance of detection as a final detection result, otherwise, performing a second instance of detection to obtain a final detection result; and (8) on the basis of a proportion of the number, which is obtained in step (7), of sites j that are determined to be MSI candidate sites in samples to be detected in the total number of sites of said sample, determining the microsatellite stability of said sample.

## Description

### FIELD OF THE DISCLOSURE

The present disclosure relates to a technical field of microsatellite detection, and specifically relates to a method for detecting microsatellite instability based on next-generation sequencing.

### BACKGROUND OF THE DISCLOSURE

Microsatellites (MS) are short tandem repeat sequences, which are widespread in human genomes. Microsatellite instability (MSI) is a phenomenon in which length alterations of MS sequence are caused by deletion or insertion mutations during DNA replication and is usually caused by deficiency of the mismatch repair (MMR).

In recent years, MSI has been found to relate to the prognosis of many types of cancer including colorectal, gastric, endometrial and ovarian cancers, etc. High levels of MSI (MSI-H) cancer patients with surgery alone, which are not applicable to some chemotherapeutic agents, such as 5-fluorouracil, exhibit an improved prognosis and better survival compared to low levels of MSI (MSI-L) cancer patients or microsatellite stable (MSS) cancer patients. In addition, studies have shown that MSI status of patients is also of great importance in guiding immunotherapy. Immunotherapy, such as PD-1 monoclonal antibody, with or without CTLA-4 monoclonal antibody, have demonstrated remarkable activity in MSI-H advanced colorectal cancer patients. In contrast, the immunotherapy is not recommended for MSS colorectal cancer patients currently. In addition to colorectal cancer, this phenomenon has been successively validated in advanced solid tumours such as gastric cancer, endometrial cancer, pancreatic cancer and bile duct cancer, etc. Therefore, MSI serves as a potential therapeutic predictor for both chemotherapy and immunotherapy, and its accurate detection of the MSI status of the patient is of great importance for clinical diagnosis, prognosis, chemotherapy sensitivity and a therapy development.

With the drastic development of high-throughput sequencing platforms, Next-generation sequencing (NGS) has been wildly used for MSI detection. NGS-based MSI detection has high throughput, high accuracy, high sensitivity and other characteristics to makes possible to detect a large amount of microsatellite sequences on genome at the same time using bioinformatic computational tools. However, conventional NGS-based MSI detection algorithms and software, such as mSINGS, MSIsensor, MSIsensor-pro, MANTIS, require a fixed negative sample reference set constructed by normal samples or MSI negative tumour samples as control. On the other hand, MSI data features of samples are highly affected to generate certain overall fluctuation due to different sequencing platforms, experimental reagents, experimental parameters, experimental batches and other processes. Therefore, in an actual test, poor detection performance of samples across batches often occurs, so that different platforms and instruments need to construct and maintain different reference sets, parameter and processes; in addition, there are significant differences in the data features of the samples between different cancer types due to the heterogeneity of cancer, so that a separate reference set normally needs to be constructed, which not only increases costs, but also brings great limitation in application, and it can not respond to data of new platforms, new instruments and new cancer types rapidly and flexibly.

Therefore, it is important to establish an efficient and convenient MSI negative sample length distribution reference set for MSI detection used across different NGS platforms, batches and cancer types.

### BRIEF SUMMARY OF THE DISCLOSURE

An objective of the present disclosure provides a method for detecting microsatellite instability based on next-generation sequencing to solve deficiencies of the existing techniques.

A technical solution of the present disclosure is as follows:
A method for detecting microsatellite instability based on next-generation sequencing, which comprises the following steps:
(1) combining sequencing data of MSI negative samples from various NGS analysis platforms, various reagent types and/or various cancer types to obtain a reference set;
(2) counting a total number of reads of MSI loci i for each of the MSI negative samples in the reference set to record as depths Di of the MSI loci i;
(3) calculating data features Fᵢ of microsatellite sequences of the MSI loci i according to a selected MSI analysis algorithm for all of the MSI loci i of each of the MSI negative samples in the reference set;
(4) counting a number of the depths Di of the MSI loci i lower than a threshold a in the entire reference set, and then selecting samples in which low-depth loci with a number of reads below the threshold a is no more than b to construct a mixed reference set;
(5) performing grouped quality control on the MSI negative samples corresponding to each of the NGS analysis platforms and each of the cancer types in the mixed reference set:
   a. for any one of the MSI loci i, obtaining a mean value MFi of the loci after removing outliers for the data features Fᵢ of all of the MSI negative samples in the same group;
   b. for each of the MSI negative samples, calculating a standard deviation StdFi of the data features Fᵢ for all of the MSI loci i, and selecting samples with the standard deviation StdFi less than a threshold c;
   c. for each of the MSI negative samples, comparing the data features Fᵢ of all of the MSI loci i with the corresponding mean value MFi, and obtaining samples that are not significantly different from the corresponding mean values MFᵢ by F-test;
   d. selecting samples in each group that synchronously satisfy requirements of the steps b and c, constructing a mixed sample reference set of each of the MSI loci i, and calculating a mean value MFi' and a standard deviation StdFi' of the data features Fᵢ for each of the MSI loci i using the mixed sample reference set;
(6) obtaining depths Dⱼ, data features Fⱼ of loci j of a test sample according to the steps (1) to (5), for the loci j of the test sample with a number of reads higher than the threshold a, if Fⱼ<MFⱼ'-x·StdFⱼ' or Fⱼ>MFⱼ'+x·StdFⱼ', then identifying the loci j as MSI candidate loci, otherwise as MSS candidate loci without considering the loci j with the number of the reads lower than the threshold a, wherein x is 1-6; and this step is a first detection;
(7) when the number of the loci j identified as the MSS candidate loci in the first detection is less than d, the result of the first detection is a final detection result; otherwise an optimal reference sample subset needs to be selected to perform a second detection for the test sample to obtain the final detection result;
(8) identifying a microsatellite stability status of the test sample by a ratio of a number of loci j identified as the MSI candidate loci in the test sample obtained in step (7) to a total number of loci in the test sample.

In a preferred embodiment of the present disclosure, the depths Di are original depths Dᵢ or effective depths Di.

Further preferably, the depths Di are the effective depths Di.

In a preferred embodiment of the present disclosure, the data features Fᵢ are a main peak depth percentage or a number of main peaks, the main peaks are any one or two of length types of the microsatellite sequences covering most of the MSI loci i.

In a preferred embodiment of the present disclosure, the threshold a is 100-300, and b is 10-30% of a total number of the MSI loci i in the sample.

In a preferred embodiment of the present disclosure, the threshold c is 0.2-0.3.

In a preferred embodiment of the present disclosure, the standard deviation StdFᵢ is the data features Fᵢ of each of the MSI negative samples in the mixed reference set divided by the mean value MFᵢ to then solved for log2 so as to get a standard deviation of the log2 value for all loci of a same MSI negative sample.

In a preferred embodiment of the present disclosure, the x is 3-5.

In a preferred embodiment of the present disclosure, the d is 5-20% of the total number of the loci of the test sample.

In a preferred embodiment of the present disclosure, the second detection comprises:
a. for the loci j identified as the MSS candidate loci in the first detection, if mathematical expectation of the data features Fⱼ of positive loci is smaller than negative loci, dividing the data features Fⱼ of the positive loci by the threshold MFⱼ'-x·StdFⱼ of the first detection for corresponding loci, then ranking result values from large to small, and retaining loci ranking at most top e, if the mathematical expectation of the data features Fⱼ of the positive loci is larger than the negative loci, dividing the data features Fⱼ of the positive loci by the threshold MFj'+x.StdFj of the first detection of the corresponding loci, then ranking result values from small to large, and retaining loci ranking at most top e to act for a subsequent matching process of an optimal negative reference subset;
b. calculating a similarity between the test sample and each of the samples in the mixed reference set based on the data features of the loci j selected in the step a in the test sample and the samples in the mixed reference set, and constructing an optimal reference subset by selecting a number of samples with highest similarity;
c. calculating a mean value MFᵢ" and a standard deviation StdFi" of the data features Fi' of each of the loci i in the optimal reference subset;
d. as shown in the step (7), then re-identifying each of the loci j of the test sample based on a threshold MFⱼ"-y·StdFⱼ" and MFⱼ"+y·StdFⱼ" of the second detection as a final detection result of each of the loci.

Further preferably, the e in the step a in the second detection is 30-50% of the total number of the loci in the test sample.

Further preferably, the method for calculating the similarity in the step b in the second detection comprises calculating the Euclidean distance, calculating the cosine distance, calculating the Manhattan distance or using a clustering method.

Further preferably, the y is 1-6.

More further preferably, the y is 3-5.

Further preferably, the e is 30-50% of the total number of the loci of the test sample.

In a preferred embodiment of the present disclosure, in the step (8), if a quotient R of the number of the loci identified as MSI divided by the total number of the MSI loci ≥ detection threshold Thr, the test sample is identified as MSI, otherwise identified as MSS.

Further preferably, the detection threshold Thr is 10-60%.

More further preferably, the detection threshold Thr 15-40%.

The present disclosure has the following advantages:
1. The present disclosure develops a method for constructing and fitting an MSI negative sample reference set based on NGS technology, and the detection method can be used to a variety of samples based on NGS sequencing for the detection of microsatellite instability status.
2. The present disclosure does not rely on the own negative control sample of the test sample or the negative sample reference set of the same batch and can automatically fit the negative reference set that is closest to its data characteristics, so as to identify MSI status of the test sample accurately, efficiently and reproducibly when the own negative sample reference set cannot be obtained.
3. The present disclosure has stronger detection performance across batches, reagents, instruments, platforms and cancer types compared with a fixed reference set;
4. The present disclosure enables substantive data characteristics between the test sample and the negative reference set to be more similar, it is not necessary to be separately optimized and adjust parameters for a type of the test sample, or even establish a separate process, so that a lot of resources and costs can be saved, and is has both theoretical and practical application values.
5. Compared with the conventional MSI detection methods, such as PCR detection and immunohistochemistry, the present disclosure has advantages of high throughput, objectivity, simple and efficient experimental solutions.

### DETAILED DESCRIPTION OF THE EMBODIMENTS

The technical solution of the present disclosure will be further clarified and described in combination with the detailed description of the embodiments.

Each of embodiments is performed according to the following method:
A method for detecting microsatellite instability based on next-generation sequencing, which comprises the following steps:
(1) combining sequencing data of MSI negative samples from various NGS analysis platforms, various reagent types and/or various cancer types to obtain a reference set;
(2) counting a total number of reads of MSI loci i for each of the MSI negative samples in the reference set to record as depths Di of the MSI loci i;
(3) calculating data features Fᵢ of microsatellite sequences of the MSI loci i according to a selected MSI analysis algorithm for all of the MSI loci i of each of the MSI negative samples in the reference set;
(4) counting a number of the depths Di of the MSI loci i lower than a threshold a in the entire reference set, and then selecting samples in which low-depth loci with a number of reads below the threshold a is no more than b to construct a mixed reference set;
(5) performing grouped quality control on the MSI negative samples corresponding to each of the NGS analysis platforms and each of the cancer types in the mixed reference set:
   a. for any one of the MSI loci i, obtaining a mean value MFi of the loci after removing outliers for the data features Fᵢ of all of the MSI negative samples in the same group;
   b. for each of the MSI negative samples, calculating a standard deviation StdFi of the data features Fᵢ for all of the MSI loci i, and selecting samples with the standard deviation StdFi less than a threshold c;
   c. for each of the MSI negative samples, comparing the data features Fᵢ of all of the MSI loci i with the corresponding mean value MFi, and obtaining samples that are not significantly different from the corresponding mean values MFi by F-test;
   d. selecting samples in each group that synchronously satisfy requirements of the steps b and c, constructing a mixed sample reference set of each of the MSI loci i, and calculating a mean value MFi' and a standard deviation StdFi' of the data features Fᵢ for each of the MSI loci i using the mixed sample reference set;
(6) obtaining depths Dⱼ, data features Fᵢ of loci j of a test sample according to the steps (1) to (5), for the loci i of the test sample with a number of reads higher than the threshold a, if Fⱼ<MFⱼ'-x·StdFⱼ' or Fⱼ>MFⱼ'+x·StdFⱼ', then identifying the loci j as MSI candidate loci, otherwise as MSS candidate loci without considering the loci j with the number of the reads lower than the threshold a, wherein x is 1-6; and this step is a first detection;
(7) if the number of the loci j identified as the MSS candidate loci in the first detection is less than d, the result of the first detection is a final detection result; otherwise an optimal reference sample subset needs to be selected to perform a second detection for the test sample to obtain the final detection result;
(8) identifying a microsatellite stability status of the test sample by a ratio of a number of loci j identified as the MSI candidate loci in the test sample obtained in step (7) to a total number of loci in the test sample.

The depths Dᵢ are original depths Dᵢ or effective depths Di, further preferably, the depths Di are the effective depths Di.

The data features Fᵢ are a main peak depth percentage or a number of main peaks, the main peaks are any one or two of length types of the microsatellite sequences covering most of the MSI loci i.

The threshold a is 100-300, and b is 10-30% of a total number of the MSI loci i in the sample.

The threshold c is 0.2-0.3.

The standard deviation StdFᵢ is the data features Fᵢ of each of the MSI negative samples in the mixed reference set divided by the mean value MFᵢ to then solved for log2 so as to get a standard deviation of the log2 value for all loci of a same MSI negative sample.

The x is 3-5.

The d is 5-20% of the total number of the loci of the test sample.

The second detection comprises:
a. for the loci j identified as the MSS candidate loci in the first detection, if mathematical expectation of the data features Fⱼ of positive loci is smaller than negative loci, dividing the data features Fⱼ of the positive loci by the threshold MFⱼ'-x·StdFⱼ of the first detection for corresponding loci, then ranking result values from large to small, and retaining loci ranking at most top e, if the mathematical expectation of the data features Fⱼ of the positive loci is larger than the negative loci, dividing the data features Fⱼ of the positive loci by the threshold MFj'+x.StdFj of the first detection of the corresponding loci, then ranking result values from small to large, and retaining loci ranking at most top e to act for a subsequent matching process of an optimal negative reference subset;
b. calculating a similarity between the test sample and each of the samples in the mixed reference set based on the data features of the loci j selected in the step a in the test sample and the samples in the mixed reference set, and constructing an optimal reference subset by selecting a number of samples with highest similarity;
c. calculating a mean value MFᵢ" and a standard deviation StdFi" of the data features Fi' of each of the loci i in the optimal reference subset;
d. as shown in the step (7), then re-identifying each of the loci j of the test sample based on a threshold MFⱼ"-y·StdFⱼ" and MFⱼ"+y·StdFⱼ" of the second detection as a final detection result of each of the loci.

The e in the step a in the second detection is 30-50% of the total number of the loci in the test sample.

The method for calculating the similarity in the step b in the second detection comprises calculating the Euclidean distance, calculating the cosine distance, calculating the Manhattan distance or using a clustering method.

The y is 1-6, and further preferably 3-5.

The e is 30-50% of the total number of the loci of the test sample.

In the step (8), if a quotient R of the number of the loci identified as MSI divided by the total number of the MSI loci ≥ detection threshold Thr, the test sample is identified as MSI, otherwise identified as MSS.

The detection threshold Thr is 10-60%, and further preferably 15-40%.

Experimental samples involved in all embodiments are all pre-tested by Sanger sequencing, loci for comparison comprises five microsatellite loci recommended by the National Cancer Institute (NCI): BAT25, BAT26, D5S346, D2S123 and D17S250. MSI-H, MSI-L and MSS status of the samples are identified by recognizing a number of altered loci. Sanger sequencing is currently a Gold Standard for the Global Goals for all genetic testing. Previous studies have shown that there is no significant difference in tumour biological characteristics of MSI-L and MSS, MSI-L and MSS samples are classified into a group of MSS samples for processing. The present disclosure classifies the MSS samples into MSS tumour tissue samples and MSS paracancerous normal samples according to sampling sites of tumour tissues. The present disclosure extracts genomic DNA from patient tissue samples, constructs libraries, amplifies, and performs NGS sequencing analysis, and specific sample information is shown in Table 1. The embodiment uses a 55-loci combination for microsatellite instability detection. For the generated sequencing data, a number of reads that covers length types of different repeat sequences of 55 loci is counted for subsequent detection and identification.

**Table 1 Sample information analysis platforms from different NGS analysis platforms, analysis models, analysis reagents and cancer types**

| | Analysis batches | Cancer types | Amount of MSI samples | Amount of MSS samples | Amount of MSS paracancerous normal samples |
|---|---|---|---|---|---|
| MiSeq-v2 | Test01-02 | CRC¹ | 6 | 31 | 26 |
| MGI200 | Test01-02 | CRC | 6 | 31 | 26 |
| NextSeq-Mid-v2.5 | Test04-05 | EC² | 24 | 62 | 43 |
| NextSeq-High-v2.5 | Test04-05 | EC | 24 | 37 | 22 |
| MiSeq-v2 | Test04-05 | EC | 19 | 7 | 43 |
| MiniSeq-High | Test01-02 | CRC | 6 | 20 | 12 |
| | | GC³ | 9 | 25 | 0 |
| | Test04-05 | EC | 12 | 30 | 31 |

| | | | | | |
|---|---|---|---|---|---|
| Note: ¹ colorectal cancer, ² endometrial cancer, ³ gastric cancer | | | | | |

Embodiment 1: Detection performance test of samples on different sequencing platforms based on a main-peak depth percentage

The test data used in the embodiment are colorectal cancer samples of the Test01-02 of the MiSeq-v2 and the MGI200 in Table 1. Data of samples of the same batch, which includes 6 Sanger-positive colorectal cancer tissue samples, 31 Sanger-negative colorectal cancer tissue samples and 26 Sanger-negative colorectal cancer paracancerous normal tissue samples, is detected by different sequencing platforms. The specific steps are as follows:

### 1.1.1 A mixed reference set is constructed

This embodiment counts numbers of reads that covers length types of different repeat sequences respectively corresponding to the 55 loci of the present disclosure in the aforementioned 114 Sanger-negative colorectal cancer samples.

For a particular microsatellite locus, based on the numbers of the reads counted above, an effective depth value and the main-peak depth percentage of each of the samples at the locus are respectively calculated. In this embodiment, the effective depth value refers to a sum of the numbers of the reads that cover all of length types of microsatellite sequences at the locus. The main peak depth percentage refers to a coverage rate of 1 or 2 of the length types of the microsatellite sequences that cover most of the locus. Specifically, when 75% of the numbers of the reads with length types ranking first in coverage are greater than the numbers of the reads with length types ranking second in the coverage, a coverage rate of the length types ranking first in the coverage is taken as the main peak depth percentage, otherwise, a sum of coverage rates of the length types ranking first and second in the coverage is taken as the main peak depth percentage; for the negative samples corresponding to each of the NGS analysis platforms (the MiSeq and the MGI200), quality control is performed according to the steps (4) and (5), specifically:
For each of the colorectal cancer negative samples, a number of loci with an effective depth value less than 300 is counted, and samples with the number > 5 are removed;
For any one of the MSI loci j, its standard score (z-score) in the negative samples is calculated based on the main peak depth percentage, 20% outliers are removed based on a value of the z-score, and a mean value MFⱼ of the main peak depth percentage of each locus is then counted;
For any one of the MSI loci j, its main peak depth percentage is divided by the mean value MFⱼ and then solved for log2 to get a log2 value logMFⱼ of the depth percentage of each locus;
For any one of the colorectal cancer negative samples, a standard deviation of each of the samples is calculated based on the logMFⱼ of each locus, and the samples with the standard deviation >0.2 are removed;
For any one of the colorectal cancer negative samples, one-way analysis of variance of the main peak depth percentage and the mean value MFⱼ of the loci are performed to obtain a significance level P of each of the test samples, and samples with P<0.05 are removed;
The negative samples after the quality control on the two sequencing platforms are then combined to obtain a final mixed reference set of the negative samples. Therefore, the mean value meanᵢ and the standard deviation stdi are calculated to obtain the main peak depth percentage of each locus.

### 1.1.2 MSI status of test samples is detected

For each of the MSI loci i of a test sample in this embodiment, the number of the reads that cover the length types of the different repeat sequences of the locus are counted, the effective depth value and the main peak depth percentage of the locus of the sample are calculated, and the loci with an effective depth no more than 300 are not considered in this embodiment;
The main peak depth percentage of each locus with the effective depth greater than 300 is compared with a corresponding threshold value meani-4-stdi of each locus calculated in the step 1.1, i.e., if the main peak depth percentage of the locus is < meani-4-stdi, the locus is then identified to be an unstable microsatellite locus, otherwise identified as a stable microsatellite locus, and a number n₁ of loci identified as MSS in the test sample is counted;
If n₁<3, a number of unstable loci of the test sample at this time is 55-m;
If n₁≥3, an optimal reference set is selected for the test sample to perform the second detection according to the step 3.2, specifically: for the loci identified as MSS in the first detection, the main peak depth percentage is divided by the threshold meani-4-stdi of the first detection of the corresponding locus, and the values are ranked from large to small, and loci at most ranked top 40 are retained. Based on the main peak depth percentage of the selected loci, the Manhattan distances between the test sample and each sample in the mixed reference set are counted, and the distance values are ranked from small to large, and the samples ranked in top 30 are selected as an optimal reference subset; at this time, the mean value mean'ᵢ and the standard deviation std'ᵢ of the main peak depth percentage of each locus in the optimal reference subset are calculated. Based on the threshold mean'ᵢ-4·std'ᵢ of the second detection, a number of loci identified as MSS in the test sample are n₂ at this time, and the number of unstable loci in the test sample is 55-n₂ at this time;
For each test sample, if the number of the unstable loci 55-n₁ or 55-n₂ is ≥15%·55, the sample is identified as MSI, and if the number is <15%.55, the sample is identified as MSS.

As there is no obvious difference in tumour biological characteristics between MSI-L and MSS, in the present disclosure, MSI-L and MSS are classified as a group of MSS, and MSI-H is classified as MSI.

### 1.1.3 MSI status of the test sample is validated

A total of 6 Sanger-positive colorectal cancer tissue samples, 31 Sanger-negative colorectal cancer tissue samples and 26 Sanger-negative colorectal cancer paracancerous normal tissue samples on the MiSeq and MGI200 sequencing platforms are tested in this embodiment.

When the 126 samples are detected using the method based on the present disclosure, the results showed that all of the 126 samples are correctly detected with 100% specificity and sensitivity;
1.2 As a comparison of this embodiment, a method based on a conventional fixed reference set is used as follows:
Comparison test 1: an own negative reference set is constructed based on the main peak depth percentage of the 57 negative colorectal cancer samples corresponding to the MiSeq sequencing platform, the mean value mean"ᵢ and the standard deviation std"ᵢ of the main peak depth percentage of each locus is calculated in the own negative reference set at this time, and 63 samples on the MiSeq sequencing platform are then detected based on the threshold value mean"ᵢ-4·std"ᵢ. The results show that all of the 63 samples are correctly detected.

Comparison test 2: As described above, 63 samples on the MGI200 sequencing platform are detected using 57 negative colorectal cancer samples corresponding to the MGI200 sequencing platform as a reference set. The results show that all of the 63 samples are also correctly detected.

Comparison test 3: As described above, 63 samples on the MiSeq sequencing platform are detected using 57 negative colorectal cancer samples corresponding to the MGI200 sequencing platform as a reference set. The results show that 6 Sanger-positive colorectal cancer tissue samples are all identified as MSI with a sensitivity of 100%, while only 7 are identified as MSS with a specificity of only 12.28% for 57 Sanger-negative samples.

Therefore, the method based on the conventional fixed reference set is not applicable for samples across different sequencing platforms.

Embodiment 2: Detection performance test of samples on different sequencing instruments based on the main peak depth percentage

The test data used in this embodiment are samples of the Test04-05 of the NextSeq and the MiSeq endometrial cancer in Table 1.

### 2.1 A method based on the present disclosure

It is data of samples of the same batch tested on a same sequencing platform (Illumina), different sequencing models (NextSeq, MiSeq), and different sequencing reagent types and versions (Mid, High, V2, V2.5). The specific steps are as follows: this embodiment constructs a mixed reference set based on 214 negative samples corresponding to the different sequencing models and the different sequencing reagent types and versions, and specific implementation steps are consistent with Embodiment 1.1. The results show that for NextSeq-Mid sequencing, all of 24 Sanger-positive samples are identified as MSI, 104 of 105 Sanger-negative samples are identified as MSS and only 1 is identified as MSI; for NextSeq-High sequencing data, all of 24 Sanger-positive samples are identified as MSI, and 58 of 59 Sanger-negative samples are identified as MSS and only 1 is identified as MSI; for MiSeq sequencing data, all of 19 Sanger-positive samples are identified as MSI, all of 50 Sanger-negative samples are identified as MSS. Overall, a sensitivity is 100%, and a specificity is 99.07%.

2.2 As a comparison of this embodiment, a method based on a conventional fixed reference set is as follows:
Comparison test 4: an own negative reference set is constructed based on the main peak depth percentage of 105 negative samples corresponding to a NextSeq-Mid-v2.5 sequencing platform, a mean value mean"ᵢ and a standard deviation std"ᵢ of the main peak depth percentage of each locus in the own negative reference set are calculated at this time, and 129 samples on the NextSeq-Mid-v2.5 sequencing platform is then detected based on the threshold value mean"ᵢ-4·std"ᵢ. The results show that 23 are identified as MSI and 1 is identified as MSS in 24 Sanger-positive samples, and 104 are identified as MSS and 1 is identified as MSI in 105 Sanger-negative samples.

Comparison test 5: as described above, 83 samples of NextSeq-High-v2.5 sequencing platform are detected using 59 negative samples corresponding to the NextSeq-High-v2.5 sequencing platform as a reference set. The results show that 23 are identified as MSI and 1 is defined as MSS in 24 Sanger-positive samples, and 58 are identified as MSS and 1 is identified as MSI in 59 Sanger-negative samples.

Comparison test 6: as described above, 69 samples on the MiSeq-v2 sequencing platform are detected using 50 negative samples corresponding to the MiSeq-v2 sequencing platform as a reference set. The results show that all of 69 samples are correctly detected.

Comparison test 7: as described above, 69 samples on the MiSeq-v2 sequencing platform are detected using 105 negative samples of the NextSeq-Mid-v2.5 sequencing platform as a reference set. The results show that of 17 are identified as MSI and 2 are identified as MSS in 19 Sanger-positive samples, and all of 50 Sanger-negative samples are identified as MSS.

It can be seen that the method based on the conventional fixed reference set, either based on a corresponding reference set or a reference set across different sequencing models, has lower sensitivity compared to the method based on the present disclosure.

Embodiment 3: Detection performance test of different cancer types of samples based on the main peak depth percentage

The test data used in this Embodiment are samples of the MiniSeq-High in Table 1. It comprises data of samples of colorectal cancer, gastric cancer and endometrial cancer detected on a same sequencing platform and instrument and respectively comprises 6 Sanger-positive colorectal cancer tissue samples, 20 Sanger-negative colorectal cancer tissue samples, 12 Sanger-negative colorectal cancer paracancerous normal tissue samples, 9 Sanger-positive gastric cancer tissue samples, 25 Sanger negative gastric cancer tissue samples, 12 Sanger-positive endometrial cancer tissue samples, 30 Sanger-negative endometrial cancer tissue samples and 31 Sanger-negative endometrial cancer paracancerous normal tissue samples.

### 3.1 Method based on the present disclosure

This embodiment constructs a mixed reference set based on the 3 cancer types of the 118 negative samples, and the specific implementation steps are consistent with Embodiment 1. The results show that a total of the 3 cancer types of 145 cases are all correctly detected with an accuracy of 100%.

3.2 As a comparison of this embodiment, a method based on a conventional fixed reference set is as follows:
Comparison test 8: an own negative reference set based on the main peak depth percentage of 32 negative colorectal cancer samples corresponding to the MiniSeq-High sequencing platform, a mean mean"ᵢ and a standard deviation std"ᵢ of the main peak depth percentage of each locus in the own negative reference set at this time, 38 colorectal cancer samples are detected on the MiniSeq-High sequencing platform according to a threshold value of mean"ᵢ-4·std"ᵢ. The results show that all of the 38 samples are correctly detected.

Comparison test 9: As described above, 25 negative gastric cancer samples corresponding to the MiniSeq-High sequencing platform are used as a reference set, and 34 gastric cancer samples on the MiniSeq-High sequencing platform are detected. The results show that all of the 34 samples are correctly detected.

Comparison test 10: As described above, 61 negative endometrial cancer samples corresponding to the MiniSeq-High sequencing platform are used as a reference set, and 73 endometrial cancer samples on the MiniSeq-High sequencing platform are detected. The results show that all of the 73 samples are correctly detected.

Comparison test 11: As described above, 32 negative colorectal cancer samples corresponding to the MiniSeq-High sequencing platform are used as a reference set, and 73 endometrial cancer samples on the MiniSeq-High sequencing platform are detected. The results show that all of 12 Sanger-positive samples are identified as MSI, and 60 of the 61 Sanger-negative samples are identified as MSS and 1 is identified as MSI.

Comparison test 12: As described above, 25 negative gastric cancer samples corresponding to the MiniSeq-High sequencing platform are used as the reference set, and 73 endometrial cancer samples on the MiniSeq-High sequencing platform are detected. The results show that all of 12 Sanger-positive samples are identified as MSI, and 60 of the 61 Sanger-negative samples are identified as MSS and 1 is identified as MSI.

Therefore, the method based on the conventional fixed reference set has limitations in an application of the reference set across different cancer types.

Embodiment 4: Detection performance test of different cancer types of samples based on a number of primary repeat units

The test data used in this embodiment is consistent with Embodiment 3, which are the different cancer types of the samples on the MiniSeq-High sequencing platform in Table 1. The specific steps are as follows:

### 4.1 A mixed reference set is constructed

This embodiment counts effective depth values and the numbers of the primary repeat units respectively corresponding to 118 Sanger-negative samples of different cancer types on the MiniSeq-High sequencing platform that cover to 55 loci of the present disclosure. In this embodiment, an effective depth value refers to a sum of numbers of reads covering all length types of microsatellite repeat sequences of the locus. The number of the primary repeat units is calculated as follows: for MSI loci i of each of the samples, length types and corresponding numbers of reads n_{(i,j)}, j=1, 2, 3..., and effective depth values nᵢ of different repeat sequences covering the locus are counted, and percentages n_{(i,j)}/nᵢ·100% of the numbers of the reads corresponding to each of the length types of the different repeat sequences in the effective depth values of the loci are calculated and ranked from large to small, the percentages are accumulated, when a sum of the percentages is >= 90%, the calculation is stopped, at this time, the number of the length types of the repeat sequences corresponding to the accumulated percentage is the number of a primary repeat unit.

For the negative samples corresponding to each of the cancer types, quality control is performed according to the steps (4) and (5), specifically:
For each of the negative samples, the number of loci with the effective depth values less than 300 are counted, and the samples with number > 5 are removed;
For any one of the MSI loci j, a standard score (z-score) in the negative samples based on the number of the primary repeat units is calculated, 20% of outliers are removed based on the z-score value, and a mean MFⱼ of the number of the primary repeat units of each of the loci is then counted;
For any one of the MSI loci j, the number of primary repeat units is divided by the mean value MFⱼ and then solved for log2 to get the log2 value logMFⱼ of the percentage of the primary repeat units of each of the loci;
For any one of the negative samples, a standard deviation of each of the samples is calculated based on the logMFⱼ of each of the loci, samples with the standard deviation > 0.2 are removed;
For any one of the negative samples, one-way analysis of variance of the number of the primary repeat units and the mean MFⱼ of the locus are performed to obtain a significance level P of each of the test samples, and samples with P<0.05 are removed;
The three different cancer types of the negative samples after separate quality control are combined to obtain a final mixed reference set of the negative samples. Therefore, the mean meanᵢ and the standard deviation stdi of the number of the primary repeat units of each locus are calculated.

### 4.1.2 MSI status of the test samples is detected

For each of the MSI loci i of a test sample in this embodiment, a number of reads that cover length types of different repeat sequences of a locus is also counted, and an effective depth value and a number of the primary repeat units of the locus of the sample are calculated, and loci with an effective depth no more than 300 are not considered in this embodiment;
The number of the primary repeat units of each locus with the effective depth greater than 300 is compared with a threshold value meanᵢ+4·stdᵢ corresponding to each locus calculated in the step 1.1, i.e., if a number of the primary repeat units of the locus is > meanᵢ+4·stdᵢ, the locus is identified to be an unstable microsatellite locus, otherwise identified as a stable microsatellite locus, and a number n₁ of loci of the test sample that is identified as MSS is counted at this time;
If n1<3, a number of unstable loci of the test sample is 55-n₁ at this time;
If n1≥3, an optimal reference set for the test sample is selected to perform a second detection according to the step 3.2, specifically: for the loci identified as the MSS in the first detection, the number of the primary repeat units is divided by the threshold value meanᵢ+4·stdᵢ of the first detection of the corresponding loci, and the values are ranked from small to large, and loci at most ranking top 40 are retained. Based on the number of the primary repeating units of the selected loci, the Manhattan distances between the test sample and each sample in the mixed reference set are counted, and the distances value are ranked from small to large, and samples at most ranking top 30 as an optimal reference subset; a mean value mean'ᵢ and a standard deviation std'ᵢ of the number of the primary repeating units of each locus in the optimal reference subset is calculated at this time. Based on a threshold mean'ᵢ+4·std'ᵢ of the second detection of the second detection, a number n₂ of loci identified as MSS in the test sample is counted at this time, and a number of unstable loci of the test sample is then 55-n₂ at this time;
For each of the test samples, if the number of unstable loci 55-n₁ or 55-n₂ is ≥ 15%-55, the sample is identified as MSI, and if the number is <15%.55, the sample is then identified as MSS.

As there is no obvious difference in tumor biological characteristics of MSI-L and MSS, the present disclosure classifies the MSI-L and the MSS as a group of MSS and classifies MSI-H as MSI.

### 4.1.3 The MSI status of the test sample is validated

Endometrial cancer samples on the MiniSeq-High sequencing platform, which comprise 12 Sanger-positive samples, 30 Sanger-negative samples and 31 Sanger-negative paracancerous normal tissue samples, are totally tested in this embodiment.

When the 73 samples are detected using the method based on the present disclosure, the results show that all of the 73 samples are correctly detected with 100% specificity and sensitivity;
4.2 As a comparison of this embodiment, the method based on the conventional fixed reference set is as follows:
Comparison test 13: an own negative reference set is constructed based on a number of primary repeat units of 61 negative endometrial cancer samples on the MiniSeq-High sequencing platform, the mean mean"ᵢ and the standard deviation std"ᵢ of the number of primary repeat units of each locus in the own negative reference set are calculated at this time, and 73 endometrial cancer samples on the MiniSeq-High sequencing platform are then detected according to the threshold value mean"ᵢ+4·std"ᵢ. The results show that all of 73 samples are correctly detected.

Comparison test 14: as described above, 73 endometrial cancer samples on the MiniSeq-High sequencing platform are detected using 32 negative colorectal cancer samples corresponding to the MiniSeq-High sequencing platform as a reference set. The results show that all of 12 Sanger-positive samples are identified as MSI, 60 of 61 Sanger-negative samples are identified as MSS, and 1 is identified as MSI.

Comparison test 15: 73 endometrial cancer samples on the MiniSeq-High sequencing platform are detected using 25 negative gastric cancer samples corresponding to the MiniSeq-High sequencing platform as a reference set. The results show that 10 of 12 Sanger-positive samples are identified as MSI and 2 are identified as MSS. 60 of 61 Sanger-negative samples are identified as MSS and 1 is identified as MSI.

It can be seen that when features based on the number of the primary repeat units are detected, the method using the conventional fixed reference set have a lower specificity compared with the method based on the present disclosure when a reference set across different cancer types is used.

In summary, the results of each of the embodiments demonstrate that the method of the present disclosure based on NGS technology constructs and selects an optimal MSI negative sample reference set of the test sample and detects the microsatellite instability status of the sample with an accuracy of 99.64% when compared with the Sanger sequencing results, it can identify the MSI status (*See* Table 2) of the sample in a more accurate and efficient way as well as have better stability across batches, reagents, instruments, platforms and cancer types compared with the method based on the conventional fixed reference set.

**Table 2 Results of microsatellite instability identification**

| | sequencing platforms/cancer types | sequencing platforms/cancer types of negative reference set | Numb er of MSI | Number of MS S | coincide nce rate with Sanger results |
|---|---|---|---|---|---|
| Embodiment 1 : | | | | | |
| Method of the present disclosure | MiSeq-v2 | matching optimal reference subset | 6(6) | 57(57) | 100% |
| | MGI200 | matching optimal reference subset | 6(6) | 57(57) | 100% |
| Comparison test 1 | MiSeq-v2 | MiSeq-v2 | 6(6) | 57(57) | 100% |
| Comparison test 2 | MGI200 | MGI200 | 6(6) | 57(57) | 100% |
| Comparison test 3 | MiSeq-v2 | MGI200 | 6(6) | 7(57) | 28.57% |

| Embodiment 2 | | | | | |
|---|---|---|---|---|---|
| Method of the present disclosure | NextSeq-Mid-v2.5 | matching optimal reference subset | 24(24) | 104(105) | 99.22% |
| | NextSeq-High-v2.5 | matching optimal reference subset | 24(24) | 58(59) | 98.8% |
| | MiSeq-v2 | matching optimal reference subset | 19(19) | 50 (50) | 100% |
| Comparison test 4 | NextSeq-Mid-v2.5 | NextSeq-Mid-v2.5 | 23(24) | 104(105) | 98.45% |
| Comparison test 5 | NextSeq-High-v2.5 | NextSeq-High-v2.5 | 23(24) | 58(59) | 97.59% |
| Comparison test 6 | MiSeq-v2 | MiSeq-v2 | 19(19) | 50(50) | 100% |
| Comparison test 7 | MiSeq-v2 | NextSeq-Mid-v2.5 | 17(19) | 50(50) | 97.1% |

| Embodiment 3 | | | | | |
|---|---|---|---|---|---|
| Method of the present disclosure | MiniSeq-High CRC¹ | matching optimal reference subset | 6(6) | 32(32) | 100% |
| | MiniSeq-High GC² | matching optimal reference subset | 9(9) | 25(25) | 100% |
| | MiniSeq-High EC³ | matching optimal reference subset | 12(12) | 61(61) | 100% |
| Comparison test 8 | MiniSeq-High CRC | MiniSeq-High CRC | 6(6) | 32(32) | 100% |
| Comparison test 9 | MiniSeq-High GC | MiniSeq-High GC | 9(9) | 25(25) | 100% |
| Comparison test 10 | MiniSeq-High EC | MiniSeq-High EC | 12(12) | 61(61) | 100% |
| Comparison test 11 | MiniSeq-High EC | MiniSeq-High CRC | 12(12) | 60(61) | 98.63% |
| Comparison test 12 | MiniSeq-High EC | MiniSeq-High GC | 12(12) | 60(61) | 98.63% |

| Embodiment 4 | | | | | |
|---|---|---|---|---|---|
| Method of the present disclosure | MiniSeq-High EC | matching optimal reference subset | 12(12) | 61(61) | 100% |
| Comparison test 13 | MiniSeq-High EC | MiniSeq-High EC | 12(12) | 61(61) | 100% |
| Comparison test 14 | MiniSeq-High EC | MiniSeq-High CRC | 12(12) | 60(61) | 98.63% |
| Comparison test 15 | MiniSeq-High EC | MiniSeq-High GC | 10(12) | 60(61) | 95.89% |

| | | | | | |
|---|---|---|---|---|---|
| Note: ¹Colorectal cancer, ²Stomach cancer, ³Endometrial cancer. | | | | | |

The aforementioned embodiments are merely some embodiments of the present disclosure, and the scope of the disclosure of is not limited thereto. Thus, it is intended that the present disclosure cover any modifications and variations provided they are made without departing from the appended claims and the specification of the present disclosure.

### INDUSTRIAL APPLICABILITY

The present disclosure discloses a method for detecting microsatellite instability based on next-generation sequencing, which comprises the following steps: (1) combining sequencing data of various MSI negative samples to obtain a reference set; (2) counting depths Di of the MSI loci i in the reference set; (3) calculating data features Fᵢ of microsatellite sequences of the MSI loci i in the MSI negative samples; (4) counting a number of the depths Di of the MSI loci i lower than a threshold a in the entire reference set, and then selecting to construct a mixed reference set; (5) performing grouped quality control on the MSI negative samples; (6) performing a first detection; (7) when the number of the loci j identified as the MSS candidate loci in the first detection is less than d, the result of the first detection is a final detection result; otherwise, a second detection is required to obtain the final detection result; (8) identifying a microsatellite stability status of the test sample by a ratio of a number of loci j identified as the MSI candidate loci in the test sample obtained in step (7) to a total number of loci in the test sample, and it has industrial applicability.

## Claims

1. A method for detecting microsatellite instability based on next-generation sequencing, **characterized in that** it comprises the following steps:
(1) combining sequencing data of MSI negative samples from various NGS analysis platforms, various reagent types and/or various cancer types to obtain a reference set;
(2) counting a total number of reads of MSI loci i for each of the MSI negative samples in the reference set to record as depths Di of the MSI loci i;
(3) calculating data features Fᵢ of microsatellite sequences of the MSI loci i according to a selected MSI analysis algorithm for all of the MSI loci i of each of the MSI negative samples in the reference set;
(4) counting a number of the depths Di of the MSI loci i lower than a threshold a in the entire reference set, and then selecting samples in which low-depth loci with a number of reads below the threshold a is no more than b to construct a mixed reference set;
(5) performing grouped quality control on the MSI negative samples corresponding to each of the NGS analysis platforms and each of the cancer types in the mixed reference set:
a. for any one of the MSI loci i, obtaining a mean value MFi of the loci after removing outliers for the data features Fᵢ of all of the MSI negative samples in the same group;
b. for each of the MSI negative samples, calculating a standard deviation StdFi of the data features Fᵢ for all of the MSI loci i, and selecting samples with the standard deviation StdFi less than a threshold c;
c. for each of the MSI negative samples, comparing the data features Fᵢ of all of the MSI loci i with the corresponding mean value MFi, and obtaining samples that are not significantly different from the corresponding mean values MFᵢ by F-test;
d. selecting samples in each group that synchronously satisfy requirements of the steps b and c, constructing a mixed sample reference set of each of the MSI loci i, and calculating a mean value MFi' and a standard deviation StdFi' of the data features Fᵢ for each of the MSI loci i using the mixed sample reference set;
(6) obtaining depths Dⱼ, data features Fⱼ of loci j of a test sample according to the steps (1) to (5), for the loci j of the test sample with a number of reads higher than the threshold a, if Fⱼ<MFⱼ'-x·StdFⱼ' or Fⱼ>MFⱼ'+x·StdFⱼ', then identifying the loci j as MSI candidate loci, otherwise as MSS candidate loci without considering the loci j with the number of the reads lower than the threshold a, wherein x is 1-6; and this step is a first detection;
(7) when the number of the loci j identified as the MSS candidate loci in the first detection is less than d, the result of the first detection is a final detection result; otherwise an optimal reference sample subset needs to be selected to perform a second detection for the test sample to obtain the final detection result;
(8) identifying a microsatellite stability status of the test sample by a ratio of a number of loci j identified as the MSI candidate loci in the test sample obtained in step (7) to a total number of loci in the test sample.

2. The method for detecting the microsatellite instability according to claim 1, **characterized in that** the depths Di are original depths Dᵢ or effective depths Di.

3. The method for detecting the microsatellite instability according to claim 2, **characterized in that** the depths Di are the effective depths Di.

4. The method for detecting the microsatellite instability according to claim 1, **characterized in that** the data features Fᵢ are a main peak depth percentage or a number of main peaks, the main peaks are any one or two of length types of the microsatellite sequences covering most of the MSI loci i.

5. The method for detecting the microsatellite instability according to claim 1, **characterized in that** the threshold a is 100-300, and b is 10-30% of a total number of the MSI loci i in the sample.

6. The method for detecting the microsatellite instability according to claim 1, **characterized in that** the threshold c is 0.2-0.3.

7. The method for detecting the microsatellite instability according to claim 1, **characterized in that** the standard deviation StdFᵢ is the data features Fᵢ of each of the MSI negative samples in the mixed reference set divided by the mean value MFᵢ to then solved for log2 so as to get a standard deviation of the log2 value for all loci of a same MSI negative sample.

8. The method for detecting the microsatellite instability according to claim 1, **characterized in that** the x is 3-5.

9. The method for detecting the microsatellite instability according to claim 1, **characterized in that** the d is 5-20% of the total number of the loci of the test sample.

10. The method for detecting the microsatellite instability according to claim 1, **characterized in that** the second detection comprises:
a. for the loci j identified as the MSS candidate loci in the first detection, if mathematical expectation of the data features Fⱼ of positive loci is smaller than negative loci, dividing the data features Fⱼ of the positive loci by the threshold MFⱼ'-x·StdFⱼ of the first detection for corresponding loci, then ranking result values from large to small, and retaining loci ranking at most top e, if the mathematical expectation of the data features Fⱼ of the positive loci is larger than the negative loci, dividing the data features Fⱼ of the positive loci by the threshold MFj'+x.StdFj of the first detection of the corresponding loci, then ranking result values from small to large, and retaining loci ranking at most top e to act for a subsequent matching process of an optimal negative reference subset;
b. calculating a similarity between the test sample and each of the samples in the mixed reference set based on the data features of the loci j selected in the step a in the test sample and the samples in the mixed reference set, and constructing an optimal reference subset by selecting a number of samples with highest similarity;
c. calculating a mean value MFᵢ" and a standard deviation StdFi" of the data features Fᵢ' of each of the loci i in the optimal reference subset;
d. as shown in the step (7), then re-identifying each of the loci j of the test sample based on a threshold MFⱼ"-y·StdFⱼ" and MFⱼ"+y·StdFⱼ" of the second detection as a final detection result of each of the loci.

11. The method for detecting the microsatellite instability according to claim 10, **characterized in that** the e in the step a in the second detection is 30-50% of the total number of the loci in the test sample.

12. The method for detecting the microsatellite instability according to claim 10, **characterized in that** the method for calculating the similarity in the step b in the second detection comprises calculating the Euclidean distance, calculating the cosine distance, calculating the Manhattan distance or using a clustering method.

13. The method for detecting the microsatellite instability according to claim 10, **characterized in that** the y is 1-6.

14. The method for detecting the microsatellite instability according to claim 13, **characterized in that** they is 3-5.

15. The method for detecting the microsatellite instability according to claim 10, **characterized in that** the e is 30-50% of the total number of the loci of the test sample.

16. The method for detecting the microsatellite instability according to claim 1, **characterized in that** in the step (8), if a quotient R of the number of the loci identified as MSI divided by the total number of the MSI loci ≥ detection threshold Thr, the test sample is identified as MSI, otherwise identified as MSS.

17. The method for detecting the microsatellite instability according to claim 16, **characterized in that** the detection threshold Thr is 10-60%.

18. The method for detecting the microsatellite instability according to claim 13, **characterized in that** the detection threshold Thr 15-40%.
